# EUROPEAN PATENT APPLICATION

(11) **EP 2 363 496 A1**
(43) Date of publication of application: **07.09.2011**
(21) Application number: 10015840.1
(22) Date of filing: 18.08.2005
(51) Int. Cl.: C12Q 1/02, G01N 33/50

(54) **Pharmacological profiling of drugs with cell-based assays**

(30) Priority: 18.08.2004 US 602317 P; 17.08.2005 US 205021
(62) Divisional of application: 05788134.4
(71) Applicant: Odyssey Thera, Inc., San Ramon, CA 94583 (US)
(72) Inventor: Westwick, John, San Ramon, CA 94583 (US); Yu, Helen, Mountain View, CA 94041 (US); MacDonald, Marnie L., Pleasanton, CA 94566 (US)
(74) Representative: Schüssler, Andrea

(57) **Abstract**

The instant invention provides a method for establishing safety profiles for chemical compounds, as well as pharmacological profiling said method comprising (A) testing the effects of said chemical compounds on the amount and/or post-translational modifications of two or more macromolecules in intact cells; (B) constructing a pharmacological profile based on the results of said tests; and (C) comparing said profile to the profile(s) of drugs with established safety characteristics. Additionally, the invention is also directed to a composition comprising an assay panel, said panel comprising at least one high-content assay for the amount and/or post-translational modification of a protein and at least one high-content assay for the amount and/or subcellular location of a protein-protein interaction.

## Description

This application claims the priority benefit under 35 U.S.C. section 119 of U.S. Provisional Patent Application No. 60/602,317 entitled "Pharmacological Profiling Of Drugs With Cell-Based Assays", filed August 18, 2004, which is in its entirety herein incorporated by reference.

### BACKGROUND OF THE INVENTION

The central challenge of the pharmaceutical industry is to develop drugs that are both safe and effective in man. Even an exquisitely selective chemical compound that binds to a therapeutic target may have completely unexpected or 'off-pathway' effects in living cells, leading to expensive pre-clinical and clinical failures. For the purposes of this invention, we define 'off-pathway' activity as any activity of a compound on a cellular target or pathway other than the intended target of the compound.

As evidenced by the 75% failure rate of drugs in clinical trials, the development of new drugs is a costly and unpredictable process, despite the number of research tools available to the pharmaceutical industry. Our central premise is that an understanding of the full spectrum of biological activities of drug candidates would help to identify potentially adverse effects of drugs prior to clinical trials. A corollary premise is that the off-pathway effects of new drugs are responsible for many if not all of the failures in new drug development.

In recent years, numerous attempts have been made to establish methods for assessing the selectivity and off-pathway activities of lead compounds. Such methods often include one or more of the following: (a) measuring the ability of a test compound of interest to bind to or inhibit purified proteins in vitro; (b) treating cells (or whole organisms) with a test compound; preparing a cell extract or lysate; and then measuring changes in the amount of various gene transcripts in the extract or lysate in response to the test compound; (c) treating cells (or whole organisms) with a test compound; preparing a cell extract or lysate; and then measuring changes in the activity, amount, or phosphorylation status of proteins in the extract or lysate in response to the test compound; or (d) preparing a cell or tissue extract or lysate, then contacting the extract or lysate with a test compound linked to a solid surface such as a bead; and identifying the proteins that bind to the test compound. Each of these approaches is described in more detail below.

In the first instance, test compounds may be individually tested against purified enzymes or receptors in vitro, to determine their ability to bind and/or inhibit proteins other than their intended targets. Methods for the measurement of drug or receptor activity are widespread and are well known to those skilled in the art. They include enzyme-linked immunoabsorbent assays; radioligand binding assays; radioactive, chemiluminescent and luminescent assays for the measurement of the products of enzyme reactions; and other biochemical techniques that vary based on the characteristics of the protein target. For example, kinases have become widespread as drug targets, and methods have been developed for assessing the selectivity of kinase inhibitors. Kinases control many important processes, including the regulation of signaling cascades within cells and have been avidly pursued as pharmaceutical targets. There are over 500 distinct kinases encoded by the human genome, making this a particularly fruitful class of targets for drug discovery. Drugs such as Gleevec™ have reached the market for the treatment of cancer, and over 20 other kinase inhibitors are in clinical trials for diseases ranging from cancer to rheumatoid arthritis. Most such compounds bind to the ATP-binding site of the kinase target. Since the ATP-binding sites of kinases are highly homologous it has been difficult to develop drug molecules that are highly specific for their intended target. As a result, a variety of companies have established kinase inhibitor profiling products and services designed to assess the selectivity of lead compounds. Widely-used profiling methods include testing of lead compounds against dozens of individual, purified kinases in vitro to determine which kinases are inhibited by the compound. Such methods are rapid, inexpensive, and increasingly comprehensive as a result of the completion of the mapping of the 'kinome' and the availability of full-length genes encoding human kinases. Providers of such profiling services and related products include ActivX Biosciences Inc., Kinexus, and PanLabs. Providers of kinase profiling products include Becton Dickinson (PowerBlot), Luminex (xMAP technology), Cell Signaling Technology, Upstate Biotechnology, Calbiochem, and a host of other commercial suppliers of reagents and instrumentation.

Such in vitro approaches have significant drawbacks with respect to pharmacological profiling. The most significant limitation is that that even a highly selective inhibitor of a kinase may be capable of binding, activating, or inhibiting a plethora of other proteins that are not kinases. Such off-target or off-pathway activities are unpredictable, and cannot be assessed in any kinase-specific assay. More to the point, it is that it is not possible to establish truly global approaches based on purified proteins, because it is simply not feasible to individually assay for each of the tens of thousands of proteins representing the biological milieu.

In this regard, methods that are capable of detecting the binding of drugs to proteins within cell or tissue lysates have an advantage over in vitro assays. High-throughput methods have been developed that involve binding the test compound to a bead or other solid surface, preparing tissue or cell extracts or lysates, and analyzing the proteins bound to the bead by mass spectroscopy, immunoprecipitation, or flow cytometry. In a second manifestation of this approach, cells or whole animals are treated with the test compound, a cell or tissue lysate is prepared, and the post-translational modification status of proteins is assessed in the lysate. The latter methodology is enabled by a rapidly expanding collection of modification-specific antibodies that bind only to the phosphorylated form of individual proteins. The proteins in the cell lysates are typically either separated by 2-dimensional gel electrophoresis and then probed using Western blotting techniques, or are analyzed by multiplexed arrays of phospho-specific antibodies on beads or on antibody arrays (e.g. Nielsen et al., 2003, PNAS 100: 9330-9335).

Methods that rely upon cell lysates often require amounts of compound that are far higher than physiological levels. More importantly, when cells or tissues are disrupted, artifacts can occur as a result of removing the proteins from their native subcellular milieu. In order to assess the mode of action of a drug within the complex biochemical pathways that make up a living cell, one needs to cast widely across the cell for drug activity.

Most pharmacological profiling is not based on protein activity but is performed with DNA microarrays (gene chips). Microarrays have spawned the field of toxicogenomics. Cells, or whole animals, are treated with the drug or compound of interest. Following a period of hours or days, messenger RNA is isolated from the cell or tissue. The pattern of expression of thousands of individual mRNAs in the absence and presence of the drug are compared. Transcriptional profiling can reveal differences between compounds, where the compounds affect the ultimate transcriptional activity of one or more pathways. Unfortunately, changes in the level of individual mRNA molecules do not always correlate directly with the level or activity of the corresponding protein at a single point in time. Furthermore, many proteins undergo numerous post-translational modifications and biomolecular interactions, which may affect the functions and activities of proteins within a tissue or cell. Thus, simply identifying all of the mRNA species present and the levels at which they are present at a particular time, may not yield the complete picture of a particular drug. Finally, although transcription reporter assays have the capacity to provide information on the response of a pathway to chemical agents, such assays only measure the consequence of pathway activation or inhibition, and not the site of action of the compound. Even a targeted and highly selective drug may affect the transcription of dozens of genes, making interpretation of the results of gene chip experiments an arduous task.

Ideally, live cells could be treated with drugs and the effect of the drug could be measured within minutes or hours at a specific point within a pathway. Unlike transcriptional reporter assays, the information obtained by monitoring an individual protein within a pathway should reflect the effect of a drug on that particular branch or node of a cell signaling pathway, not its endpoint. Unlike drug profiling performed with cell lysates, the use of intact cells would enable studies of physiologically relevant concentrations of drugs. Therefore, we sought to establish a strategy and methodology for global pharmacological profiling in intact cells. Ideally such a methodology would have the following attributes: (a) the method would be applicable to intact cells or tissues, not requiring cell lysis; (b) the method could be applied to any drug class, target class, or drug mechanism of action; (c) the method would be capable of providing fine detail of the mechanism of action of the drug of interest; (d) the method would be amenable to large-scale automated analyses using off-the-shelf instrumentation. In particular we sought to determine whether direct measures of signaling events in intact human cells could be used for pharmacological profiling.

The background for the present invention is as follows. Binding of agonists to receptors induces a cascade of intracellular events mediated by other signaling molecules. These events cause a coordinated cascade of intracellular events that influences the behavior of the living cell. Often, post-translational modifications of particular proteins or other macromomolecules occur dynamically upon addition of an agonist, an antagonist or an inhibitor of a pathway. Frequently, such signaling cascades involve cycles of post-translational modifications of proteins, such as phosphorylation and dephosphorylation by kinases and phosphatases, respectively. These events are carried out by distinct protein kinases, which phosphorylate other proteins on serine, threonine or tyrosine residues. In turn, protein phosphatases are responsible for dephosphorylating other proteins. Phospho-specific antibodies allow for the detection of the net changes in phosphorylation status that result from phosphorylation and dephosphorylation of proteins. Such antibodies have become standard reagents in research laboratories, and are used in conjunction with a number of in vitro methods that include Western blotting, immunoprecipitation, ELISA (enzyme-linked immunoabsorbent assays), and multiplexed bead assays. A variety of commercial entities sell such antibodies, including Bio-Rad Laboratories; Cell Signaling Technology; Calbiochem; and Becton-Dickinson. Such antibodies can be used to analyze intact cells by flow cytometry and by immunofluorescence.

Phospho-specific antibodies have been applied to a variety of research investigations of individual signaling proteins and pathways. The vast majority of these studies involve cell or tissue lysates. The prior art is remarkably silent on pharmacological profiling in intact cells. For the purposes of the present invention we focus on methods that enable the quantification and/or localization of proteins in intact cells. In particular, for the purposes of drug discovery we focus on pharmacological profiling in human cells. A preferred embodiment of the current invention uses immunofluorescence assays in human cells in combination with high-content imaging systems and/or automated microscopy.

### OBJECTS AND ADVANTAGES OF THE INVENTION

- It is an object of the present invention to provide a method for pharmacological profiling of drugs, drug candidates, and drug leads on a genome-wide scale.
- It is a further object of the invention to provide methods for assessing the activity, specificity, potency, time course, and mechanism of action of chemical compounds on a broad scale.
- It is also an object of the invention to allow determination of the selectivity of a chemical compound within the context of a living cell.
- It is an additional object of the present invention to allow detection of the potential off-pathway effects, adverse effects, or toxic effects of a chemical compound within the biological context of a cell of interest.
- It is an additional object of the invention to enable lead optimization, by performing pharmacological profiling of a collection or a series of lead compounds in an iterative manner until a desired pharmacological profile is obtained.
- A further object of the invention is to enable attrition of drug candidates with undesirable or toxic properties.
- It is a further object of the invention to establish pre-clinical safety profiles for new drug candidates.
- It is a further object of the present invention to improve the efficiency of the drug discovery process by identifying unintended effects of lead compounds prior to clinical trials.
- It is a further object of the present invention to improve the safety of first-in-class drugs by identifying adverse, toxic or other off-pathway effects prior to clinical trials.
- It is an additional object of the present invention to identify positive or negative effects of drug excipients, carriers or drug delivery agents.
- It is a further object of the present invention to provide methods suitable for the development of 'designer drugs' with predetermined properties.
- An additional object of the invention is to enable the identification of new therapeutic indications for known drugs.
- Another object of this invention is to provide a method for analyzing the activity of any class of pharmacological agent on any biochemical pathway.
- A further object of this invention is to enable the identification of the biochemical pathways underlying drug toxicity.
- A further object of this invention is to enable the identification of the biochemical pathways underlying drug efficacy for a broad range of diseases.
- A further object of this invention is to provide methods, assays and compositions useful for drug discovery and evaluation.
- An additional object of the invention is to provide panels of assays suitable for pharmacological profiling.
- The present invention has the advantage of being broadly applicable to any pathway, gene, gene library, drug target class, reporter protein, detection mode, synthetic or natural product, chemical entity, assay format, automated instrumentation, or cell type of interest.

### SUMMARY OF THE INVENTION

The present invention seeks to fulfill the above-mentioned needs for pharmaceutical discovery. The present invention teaches that cell-based assays can be used to identify the mechanism of action, selectivity, and adverse or off-pathway effects of pharmacologically active agents. The present invention provides a general strategy for carrying out drug analysis and pharmacological profiling based on cell-based assays. A preferred embodiment of the present invention comprises high-content assays in intact cells. The novel methodology of this invention enables: (1) Direct visualization of the molecular architecture of specific cellular responses at the level of the discrete molecules that enable such cellular architecture; (2) Direct and quantitative analysis of drug effects on cellular signaling networks in a manner never before possible; and (3) The creation of quantitative and predictive pharmacological profiles of lead compounds and drugs regardless of their mechanisms of action.

Cellular responses are mediated by complex networks of proteins that are resident within subcellular compartments. Cell proliferation, cell-death (apoptosis), chemotaxis, metastases etc. are all controlled at the level of the proteins that act in concert to regulate cell behavior. This invention allows the quantitative analysis of the effects of chemical compounds on biochemical networks on a large scale. Importantly, the present invention is directed to a wide spectrum of chemical structures and drug targets, providing an advantage over previous methods that are limited to kinases. The invention enables an analysis of the spectrum of activity of any chemical compound, for any known or novel drug class or target class, and for chemical compounds with completely unknown mechanisms of action. We teach that any class of drug or target can be profiled using cell-based assays for post-translational modifications of macromolecules (proteins). Drug target classes that can be studied with the present invention include G-protein coupled receptors, growth factor receptors, protease inhibitors, nuclear hormone receptors, membrane hormone receptors, kinases, phosphatases, hydrolases, proteasome inhibitors, and any other known target class. If the target or mechanism of action of the compound of interest is not known, the present methodology will enable identification of the mechanism of action.

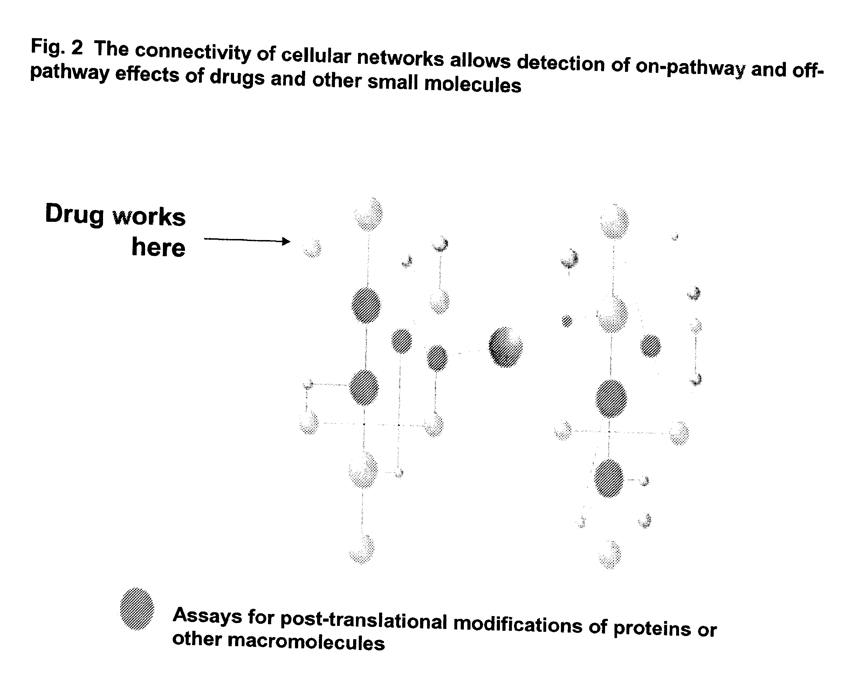

The principle of the invention relies on the connectivity of cellular networks, such that action of a drug at a particular point in a pathway can be measured by a measurable alteration in the post-translational modification status of macromolecules downstream - but physically linked to - the drug target. For example, stimulation of a canonical signal transduction pathway by a pathway agonist often leads to the phosphorylation of key proteins that participate in that pathway. The effect of a drug could therefore be assessed by quantifying the amount and/or location of two or more phospho-proteins in the absence and presence of the pathway agonist. Thus the phospho-proteins serve as sentinels of pathway activity. For example, a drug acting upstream of a sentinel would block or inhibit the phosphorylation of the sentinel in response to a cellular stimulus (see Fig 2). Thus, the phosphorylation status of the phosphoprotein in the absence or presence of a chemical compound can reveal whether or not the test compound acts on that pathway, thereby providing information on drug selectivity. This principle enables a single assay to potentially report on dozens of events in the intact cell. This also means that it is not necessary to construct an assay for every protein that may be affected by the drug of interest. By combining multiple assays (sentinels) in a panel, the full spectrum of activity can be identified; the profile of activity can be compared with that of known drugs; and lead compounds can be attrited based on undesirable profiles.

The invention requires a direct method for quantifying and/or localizing a modified protein in an intact cell. The present invention requires that compounds of interest are tested against a panel of cellular assays in order to obtain profiles of their activities. A wide variety of antibodies, probes, and stains can be employed in conjunction with the invention. Examples of suitable antibodies are shown in Table 1. These and other antibodies or targeted probes can be used in conjunction with a wide variety of biological dyes or stains, including stains of subcellular compartments (nucleus, membrane, cytosol, mitochondria, golgi, etc.); ion-sensitive dyes such as calcium-sensitive dyes; dyes that measure apoptosis or changes in cell cycle state; DNA intercalating dyes; and other commonly used biochemical and cell biological reagents. For example, co-staining of subcellular compartments would allow the fine details of the effects of drugs to be assessed. Such biochemical reagents and methods for their use are well known to those skilled in the art.

In addition to phospho-specific antibodies, other modification-state-specific antibodies can, in principle, be generated for any macromolecule that undergoes a post-translational modification in the cell. Such novel reagents can be used in conjunction with this invention. Such post-translational modifications include methylation, acetylation, famesylation, glycosylation, myristylation, ubiquitination, sumoylation, and other modifications. Such alterations may be detected using antibodies in conjunction with immunofluorescence, as described herein; however, the method is not limited to the use of antibodies. It is important to note that the invention is not limited to specific reagents or classes or reagents, or protocols for their use. Alternative (non-antibody) probes of target or pathway activity can be used, so long as they (a) bind differentially upon a change in a macromolecule in a cell, such that they reflect a change in pathway activity, cell signaling, or cell state related to the effect of a drug; (b) can be washed out of the cell in the unbound state, so that bound probe can be detected over the unbound probe background; and (c) can be detected either directly or indirectly, e.g. with a fluorescent or luminescent method. A variety of organic molecules, peptides, ligands, natural products, nucleosides and other probes can be detected directly, for example by labeling with a fluorescent or luminescent dye or a quantum dot; or can be detected indirectly, for example, by immunofluorescence with the aid of an antibody that recognizes the probe when it is bound to its target. Such probes could include ligands, native or non-native substrates, competitive binding molecules, peptides, nucleosides, and a variety of other probes that bind differentially to their targets based on post-translational modification states of the targets. It will be appreciated by one skilled in the art that some methods and reporters will be better suited to different situations. Particular reagents, fixing and staining methods may be more or less optimal for different cell types and for different pathways or targets.

**TABLE 1. Examples of targeted reagents that may be used in conjunction with the present invention**

| |
|---|
| Akt (pS472/pS473), Phospho-Specific (PKBa) Antibodies |
| Caveolin (pY14), Phospho-Specific Antibodies |
| Cdkl/Cdc2 (pY15), Phospho-Specific Antibodies |
| eNOS (pS1177), Phospho-Specific Antibodies |
| eNOS (pT495), Phospho-Specific Antibodies |
| ERK1/2 (pT202/pY204), Phospho-Specific Antibodies (p44/42 MAPK) |
| FAK (pY397), Phospho-Specific Antibodies |
| IkBa (pS32/pS36), Phospho-Specific Antibodies |
| Integrin b3 (pY759), Phospho-Specific Antibodies |
| JNK (pT183/pY185), Phospho-Specific Antibodies |
| Lck (pY505), Phospho-Specific Antibodies |
| p38 MAPK (pT180/pY182), Phospho-Specific Antibodies |
| p120 Catenin (pY228), Phospho-Specific Antibodies |
| p120 Catenin (pY280), Phospho-Specific Antibodies |
| p120 Catenin (pY96), Phospho-Specific Antibodies |
| Paxillin (pY118), Phospho-Specific Antibodies |
| Phospholipase Cg (pY783), Phospho-Specific Antibodies |
| PKARIIb (pS114), Phospho-Specific Antibodies |
| 14-3-3 Binding Motif Phospho-specific Antibodies |
| 4E-BP1 Phospho-specific Antibodies |
| AcCoA Carboxylase (Acetyl CoA) Phospho-specific Antibodies |
| Adducin Phospho-specific Antibodies |
| AFX Phospho-specific Antibodies |
| AIK (Aurora 2) Phospho-specific Antibodies |
| Akt (PKB) Phospho-specific Antibodies |
| Akt (PKB) Substrate Phospho-specific Antibodies |
| ALK Phospho-specific Antibodies |
| AMPK alpha Phospho-specific Antibodies |
| AMPK beta1 Phospho-specific Antibodies |
| APP Phospho-specific Antibodies |
| Arg-X-Tyr/Phe-X-pSer Motif Phospho-specific Antibodies |
| Arrestin 1, beta Phospho-specific Antibodies |
| ASK1 Phospho-specific Antibodies |
| ATF-2 Phospho-specific Antibodies |
| ATM / ATR Substrate Phospho-specific Antibodies |
| Aurora 2 (AIK) Phospho-specific Antibodies |
| Bad Phospho-specific Antibodies |
| Bcl-2 Phospho-specific Antibodies |
| Bcr Phospho-specific Antibodies |
| Bim EL Phospho-specific Antibodies |
| BLNK Phospho-specific Antibodies |
| BMK1 (ERK5) Phospho-specific Antibodies |
| BRCA1 Phospho-specific Antibodies |
| Btk Phospho-specific Antibodies |
| C/EBP alpha Phospho-specific Antibodies |
| C/EBP beta Phospho-specific Antibodies |
| c-Abl Phospho-specific Antibodies |
| CAKb Phospho-specific Antibodies |
| Caldesmon Phospho-specific Antibodies |
| CaM Kinase II Phospho-specific Antibodies |
| Cas, p130 Phospho-specific Antibodies |
| Catenin, beta Phospho-specific Antibodies |
| Catenin, p120 Phospho-specific Antibodies |
| Caveolin 1 Phospho-specific Antibodies |
| Caveolin 2 Phospho-specific Antibodies |
| Caveolin Phospho-specific Antibodies |
| c-Cbl Phospho-specific Antibodies |
| CD117 (c-Kit) Phospho-specific Antibodies |
| CD19 Phospho-specific Antibodies |
| cdc2 p34 Phospho-specific Antibodies |
| cdc2 Phospho-specific Antibodies |
| cdc25 C Phospho-specific Antibodies |
| cdk1 Phospho-specific Antibodies |
| cdk2 Phospho-specific Antibodies |
| CDKs Substrate Phospho-specific Antibodies |
| CENP-A Phospho-specific Antibodies |
| c-erbB-2 Phospho-specific Antibodies |
| Chk1 Phospho-specific Antibodies |
| Chk2 Phospho-specific Antibodies |
| c-Jun Phospho-specific Antibodies |
| c-Kit (CD117) Phospho-specific Antibodies |
| c-Met Phospho-specific Antibodies |
| c-Myc Phospho-specific Antibodies |
| Cofilin 2 Phospho-specific Antibodies |
| Cofilin Phospho-specific Antibodies |
| Connexin 43 Phospho-specific Antibodies |
| Cortactin Phospho-specific Antibodies |
| CPI-17 Phospho-specific Antibodies |
| cPLA2 Phospho-specific Antibodies |
| c-Raf (Raf1) Phospho-specific Antibodies |
| CREB Phospho-specific Antibodies |
| c-Ret Phospho-specific Antibodies |
| CrkII Phospho-specific Antibodies |
| CrkL Phospho-specific Antibodies |
| Cyclin B1 Phospho-specific Antibodies |
| DARPP-32 Phospho-specific Antibodies |
| DNA-topoisomerase II alpha Phospho-specific Antibodies |
| Dok-2, p56 Phospho-specific Antibodies |
| eEF2 Phospho-specific Antibodies |
| eEF2k Phospho-specific Antibodies |
| EGF Receptor (EGFR) Phospho-specific Antibodies |
| eIF2 alpha Phospho-specific Antibodies |
| eIF2B epsilon Phospho-specific Antibodies |
| eIF4 epsilon Phospho-specific Antibodies |
| eIF4 gamma Phospho-specific Antibodies |
| Elk-1 Phospho-specific Antibodies |
| eNOS Phospho-specific Antibodies |
| EphA3 Phospho-specific Antibodies |
| Ephrin B Phospho-specific Antibodies |
| erbB-2 Phospho-specific Antibodies |
| ERK1 / ERK2 Phospho-specific Antibodies |
| ERK5 (BMK1) Phospho-specific Antibodies |
| Estrogen Receptor alpha (ER-a) Phospho-specific Antibodies |
| Etk Phospho-specific Antibodies |
| Ezrin Phospho-specific Antibodies |
| FADD Phospho-specific Antibodies |
| FAK Phospho-specific Antibodies |
| FAK2 Phospho-specific Antibodies |
| Fc gamma RIIb Phospho-specific Antibodies |
| FGF Receptor (FGFR) Phospho-specific Antibodies |
| FKHR Phospho-specific Antibodies |
| FKHRL1 Phospho-specific Antibodies |
| FLT3 Phospho-specific Antibodies |
| FRS2-alpha Phospho-specific Antibodies |
| Gab1 Phospho-specific Antibodies |
| Gab2 Phospho-specific Antibodies |
| GABA B Receptor Phospho-specific Antibodies |
| GAP-43 Phospho-specific Antibodies |
| GATA4 Phospho-specific Antibodies |
| GFAP Phospho-specific Antibodies |
| Glucocorticoid Receptor Phospho-specific Antibodies |
| GluR1 (Glutamate Receptor 1) Phospho-specific Antibodies |
| GluR2 (Glutamate Receptor 2) Phospho-specific Antibodies |
| Glycogen Synthase Phospho-specific Antibodies |
| GRB10 Phospho-specific Antibodies |
| GRK2 Phospho-specific Antibodies |
| GSK-3 alpha / beta Phospho-specific Antibodies |
| GSK-3 alpha Phospho-specific Antibodies |
| GSK-3 beta (Glycogen Synthase Kinase) Phospho-specific Antibodies |
| GSK-3 beta Phospho-specific Antibodies |
| GSK-3 Phospho-specific Antibodies |
| H2A.X Phospho-specific Antibodies |
| Hck Phospho-specific Antibodies |
| HER-2 (ErbB2) Phospho-specific Antibodies |
| Histone H1 Phospho-specific Antibodies |
| Histone H2A.X Phospho-specific Antibodies |
| Histone H2B Phospho-specific Antibodies |
| Histone H3 Phospho-specific Antibodies |
| HMGN1 (HMG-14) Phospho-specific Antibodies |
| Hsp27 (Heat Shock Protein 27) Phospho-specific Antibodies |
| IkBa (I kappa B-alpha) Phospho-specific Antibodies |
| Integrin alpha-4 Phospho-specific Antibodies |
| Integrin beta-1 Phospho-specific Antibodies |
| Integrin beta-3 Phospho-specific Antibodies |
| IR (Insulin Receptor) Phospho-specific Antibodies |
| IR / IGF1R (Insulin/Insulin-Like Growth Factor-1 Receptor) Phospho-specific Antibodies |
| IRS-1 Phospho-specific Antibodies |
| IRS-2 Phospho-specific Antibodies |
| Jak1 Phospho-specific Antibodies |
| Jak2 Phospho-specific Antibodies |
| JNK (SAPK) Phospho-specific Antibodies |
| Jun Phospho-specific Antibodies |
| KDR Phospho-specific Antibodies |
| Keratin 18 Phospho-specific Antibodies |
| Keratin 8 Phospho-specific Antibodies |
| Kinase Substrate Phospho-specific Antibodies |
| Kip1, p27 Phospho-specific Antibodies |
| LAT Phospho-specific Antibodies |
| Lck Phospho-specific Antibodies |
| Leptin Receptor Phospho-specific Antibodies |
| LKB1 Phospho-specific Antibodies |
| Lyn Phospho-specific Antibodies |
| MAP Kinase / CDK Substrate Phospho-specific Antibodies |
| MAP Kinase, p38 Phospho-specific Antibodies |
| MAP Kinase, p44 /42 Phospho-specific Antibodies |
| MAPKAP Kinase 1a (Rsk1) Phospho-specific Antibodies |
| MAPKAP Kinase 2 Phospho-specific Antibodies |
| MARCKS Phospho-specific Antibodies |
| Maturation Promoting Factor (MPF) Phospho-specific Antibodies |
| M-CSF Receptor Phospho-specific Antibodies |
| MDM2 Phospho-specific Antibodies |
| MEK1 / MEK2 Phospho-specific Antibodies |
| MEK1 Phospho-specific Antibodies |
| MEK2 Phospho-specific Antibodies |
| MEK4 Phospho-specific Antibodies |
| MEK7 Phospho-specific Antibodies |
| Met Phospho-specific Antibodies |
| MKK3 / MKK6 Phospho-specific Antibodies |
| MKK4 (SEK1) Phospho-specific Antibodies |
| MKK7 Phospho-specific Antibodies |
| MLC Phospho-specific Antibodies |
| MLK3 Phospho-specific Antibodies |
| Mnk1 Phospho-specific Antibodies |
| MPM2 Phospho-specific Antibodies |
| MSK1 Phospho-specific Antibodies |
| mTOR Phospho-specific Antibodies |
| Myelin Basic Protein (MBP) Phospho-specific Antibodies |
| Myosin Light Chain 2 Phospho-specific Antibodies |
| MYPT1 Phospho-specific Antibodies |
| neu (Her2) Phospho-specific Antibodies |
| Neurofilament Phospho-specific Antibodies |
| NFAT1 Phospho-specific Antibodies |
| NF-kappa B p65 Phospho-specific Antibodies |
| Nibrin (p95 / NBS1) Phospho-specific Antibodies |
| Nitric Oxide Synthase, Endothelial (eNOS) Phospho-specific Antibodies |
| Nitric Oxide Synthase, Neuronal (nNOS) Phospho-specific Antibodies |
| NMDA Receptor 1 (NMDAR1) Phospho-specific Antibodies |
| NMDA Receptor 2B (NMDA NR2B) Phospho-specific Antibodies |
| nNOS Phospho-specific Antibodies |
| NPM Phospho-specific Antibodies |
| Opioid Receptor, delta Phospho-specific Antibodies |
| Opioid Receptor, mu Phospho-specific Antibodies |
| p53 Phospho-specific Antibodies |
| PAK1 / 2 / 3 Phospho-specific Antibodies |
| PAK2 Phospho-specific Antibodies |
| Paxilin Phospho-specific Antibodies |
| Paxillin Phospho-specific Antibodies |
| PDGF Receptor alpha / beta Phospho-specific Antibodies |
| PDGF Receptor alpha Phospho-specific Antibodies |
| PDGF Receptor beta Phospho-specific Antibodies |
| PDGFRb (Platelet Derived Growth Factor Receptor beta) Phospho-specific Antibodies |
| PDK1 Docking Motif Phospho-specific Antibodies |
| PDK1 Phospho-specific Antibodies |
| PDK1 Substrate Phospho-specific Antibodies |
| PERK Phospho-specific Antibodies |
| PFK-2 Phospho-specific Antibodies |
| Phe Phospho-specific Antibodies |
| Phospholamban Phospho-specific Antibodies |
| Phospholipase C gamma-1 Phospho-specific Antibodies |
| Phosphotyrosine IgG Phospho-specific Antibodies |
| phox, p40 Phospho-specific Antibodies |
| PI3K Binding Motif, p85 Phospho-specific Antibodies |
| Pin1 Phospho-specific Antibodies |
| PKA Substrate Phospho-specific Antibodies |
| PKB (Akt) Phospho-specific Antibodies |
| PKB (Akt) Substrate Phospho-specific Antibodies |
| PKC alpha / beta II Phospho-specific Antibodies |
| PKC alpha Phospho-specific Antibodies |
| PKC delta / theta Phospho-specific Antibodies |
| PKC delta Phospho-specific Antibodies |
| PKC epsilon Phospho-specific Antibodies |
| PKC eta Phospho-specific Antibodies |
| PKC gamma Phospho-specific Antibodies |
| PKC Phospho-specific Antibodies |
| PKC Substrate Phospho-specific Antibodies |
| PKC theta Phospho-specific Antibodies |
| PKC zeta / lambda Phospho-specific Antibodies |
| PKD (PKC mu) Phospho-specific Antibodies |
| PKD2 Phospho-specific Antibodies |
| PKR Phospho-specific Antibodies |
| PLC beta 3 Phospho-specific Antibodies |
| PLC gamma 1 Phospho-specific Antibodies |
| PLC gamma 2 Phospho-specific Antibodies |
| PLD1 Phospho-specific Antibodies |
| PP1 alpha Phospho-specific Antibodies |
| PP2A Phospho-specific Antibodies |
| PPAR Alpha Phospho-specific Antibodies |
| PRAS40 Phospho-specific Antibodies |
| Presenilin-2 Phospho-specific Antibodies |
| PRK2 (pan-PDK1 phosphorylation site) Phospho-specific Antibodies |
| Progesterone Receptor Phospho-specific Antibodies |
| Protein Kinase A, RII (PKARII) Phospho-specific Antibodies |
| Protein Kinase B Phospho-specific Antibodies |
| Protein Kinase B Substrate Phospho-specific Antibodies |
| Protein Kinase C, alpha (PKCa) Phospho-specific Antibodies |
| Protein Kinase C, epsilon (PKCe) Phospho-specific Antibodies |
| PTEN Phospho-specific Antibodies |
| Pyk2 Phospho-specific Antibodies |
| Rac1 / cdc42 Phospho-specific Antibodies |
| Rac-Pk Phospho-specific Antibodies |
| Rac-Pk Substrate Phospho-specific Antibodies |
| Rad 17 Phospho-specific Antibodies |
| Rad17 Phospho-specific Antibodies |
| Raf-1 Phospho-specific Antibodies |
| Ras-GRF1 Phospho-specific Antibodies |
| Rb (Retinoblastoma Protein) Phospho-specific Antibodies |
| Ret Phospho-specific Antibodies |
| Ribosomal Protein S6 Phospho-specific Antibodies |
| RNA polymerase II Phospho-specific Antibodies |
| Rsk, p90 Phospho-specific Antibodies |
| Rsk1 (MAPKAP K1a) Phospho-specific Antibodies |
| Rsk3 Phospho-specific Antibodies |
| S6 Kinase Phospho-specific Antibodies |
| S6 Kinase, p70 Phospho-specific Antibodies |
| S6 peptide Substrate Phospho-specific Antibodies |
| SAPK (JNK) Phospho-specific Antibodies |
| SAPK2 (Stress-activated Protein Kinase, SKK3, MKK3) Phospho-specific Antibodies |
| SEK1 (MKK4) Phospho-specific Antibodies |
| Serotonin N-AT Phospho-specific Antibodies |
| Serotonin-N-AT Phospho-specific Antibodies |
| SGK Phospho-specific Antibodies |
| Shc Phospho-specific Antibodies |
| SHIP Phospho-specific Antibodies |
| SHP-2 Phospho-specific Antibodies |
| SLP-76 Phospho-specific Antibodies |
| Smad1 Phospho-specific Antibodies |
| Smad2 Phospho-specific Antibodies |
| SMC1 Phospho-specific Antibodies |
| SMC3 Phospho-specific Antibodies |
| SOX-9 Phospho-specific Antibodies |
| Src Family Negative Regulatory Site Phospho-specific Antibodies |
| Src Family Phospho-specific Antibodies |
| Src Phospho-specific Antibodies |
| Stat1 Phospho-specific Antibodies |
| Stat2 Phospho-specific Antibodies |
| Stat3 Phospho-specific Antibodies |
| Stat4 Phospho-specific Antibodies |
| Stat5 Phospho-specific Antibodies |
| Stat5A / Stat5B Phospho-specific Antibodies |
| Stat5ab Phospho-specific Antibodies |
| Stat6 Phospho-specific Antibodies |
| Syk Phospho-specific Antibodies |
| Synapsin Phospho-specific Antibodies |
| Synapsin site 1 Phospho-specific Antibodies |
| Tau Phospho-specific Antibodies |
| Tie 2 Phospho-specific Antibodies |
| Trk A Phospho-specific Antibodies |
| Troponin I, Cardiac Phospho-specific Antibodies |
| Tuberin Phospho-specific Antibodies |
| Tyk 2 Phospho-specific Antibodies |
| Tyrosine Hydroxylase Phospho-specific Antibodies |
| Tyrosine Phospho-specific Antibodies |
| VASP Phospho-specific Antibodies |
| Vav1 Phospho-specific Antibodies |
| Vav3 Phospho-specific Antibodies |
| VEGF Receptor 2 Phospho-specific Antibodies |
| Zap-70 Phospho-specific Antibodies |

The present invention is not limited to the type of cell or tissue chosen for the analysis. The cell type can be a human cell, a mammalian cell (mouse, monkey, hamster, rat, rabbit or other species), a plant protoplast, yeast, fungus, or any other cell type of interest. The cell can also be a cell line or a primary cell. Human cells are preferred for the purposes of drug discovery, but mammalian cells can also be used. The cell can be a component of an intact tissue or animal, or in the whole body; or can be isolated from a biological sample or organ. For example, the present invention can be used in fungal cells to identify antifungal agents that block key pathways; or in plant cells to identify chemical agents that stimulate growth-related pathways or that block disease pathways. Importantly, the present invention can be used in mammalian or human cells to identify agents that block disease-related pathways and do not have off-pathway or adverse effects, thereby allowing early predictions of selectivity and allowing the development of predictive models of clinical safety. The present invention can be used in conjunction with drug discovery for any disease of interest including cancer, diabetes, cardiovascular disease, inflammation, neurodegenerative diseases, and other chronic or acute diseases afflicting mankind.

The present invention can be used in intact cells or tissues in any milieu, context or system. This includes cells in culture, organs in culture, and in live organisms. For example, this invention can be used in model organisms such as Drosophila or zebrafish. This invention can be used in preclinical studies, for example in mice. Mice can be treated with a drug and then a variety of cells or tissues can be harvested and used to construct immunofluorescence assays. This invention can also be used in nude mice, for example, human cells can be implanted as xenografts in nude mice, and a drug or other compound administered to the mouse. Cells can then be re-extracted from the implant and used for pharmacological profiling.

Any type of drug lead or other chemical compound of interest can be profiled with the methods provided herein. Such compounds include synthetic molecules, natural products, combinatorial libraries, known or putative drugs, ligands, antibodies, peptides, small interfering RNAs (siRNAs), or any other chemical agent whose activity is desired to be tested. Screening hits from combinatorial library screening or other high-throughput screening campaigns can be used in conjunction with the present invention. The invention can be used to identify those compounds with more desirable properties as compared with those compounds with less desirable properties. Therefore the present invention is suitable for use in optimization and/or attrition of lead compounds with unexpected, undesirable, or toxic properties.

In the case of an increase or decrease in the amount of a signal in response to a chemical agent, the bulk fluorescent or luminescent signal can be quantified. In the event of a change in the subcellular location of a signal in response to drug, cells are imaged by automated microscopy or image analysis and the sub-cellular location of the signal is detected and quantified. Proprietary and non-proprietary algorithms suitable for conversion of pixel intensity to subcellular location have been described; such software is often sold in conjunction with commercially available instrumentation systems. Any such algorithms, software and hardware can be used in conjunction with this invention.

Some proteins are not modified post-translationally, or, are modified constitutively - that is, their modifications do not change in response to external stimuli, environmental conditions, or other perturbants. By 'respond' we mean that a particular protein undergoes a change in modification status and/or subcellular distribution in response to a perturbation. Other post-translational modifications do respond and are induced by binding of an agonist, hormone or growth factor to a receptor which induces a signaling cascade or by a small molecule that activates an intracellular protein or enzyme. Other modifications can be inhibited, for example by binding of an antagonist or an antibody to a receptor thereby blocking a signaling cascade; by an siRNA, which silences a gene coding for a protein that is critical for a pathway; or by a drug that inhibits a particular protein within a pathway. These examples and the methods provided herein are meant to illustrate the breadth of the invention and are not limiting for the practice of the invention.

The methods and assays provided herein may be performed in multiwell formats, in microtiter plates, in multispot formats, or in arrays, allowing flexibility in assay formatting and miniaturization. The choices of assay formats and detection modes are determined by the biology of the process and the functions of the proteins within the complex being analyzed. It should be noted that in either case the assays that are the subject of the present invention can be read with any instrument that is suitable for detection of the signal that is generated by the chosen reporter. Luminescent, fluorescent or bioluminescent signals are easily detected and quantified with any one of a variety of automated and/or high-throughput instrumentation systems including fluorescence multi-well plate readers, fluorescence activated cell sorters (FACS) and automated cell-based imaging systems. The latter systems allow for spatial resolution of the signal. A variety of instrumentation systems have been developed to automate high-content assays including the automated fluorescence imaging and automated microscopy systems developed by Cellomics, Amersham (GE Medical Systems), Q3DM (Beckman Coulter), Evotec GmbH, Universal Imaging (Molecular Devices), Atto (Becton Dickinson) and Zeiss. Fluorescence recovery after photobleaching (FRAP) and time lapse fluorescence microscopy have also been used to study protein mobility in living cells.

### BRIEF DESCRIPTION OF THE DRAWINGS

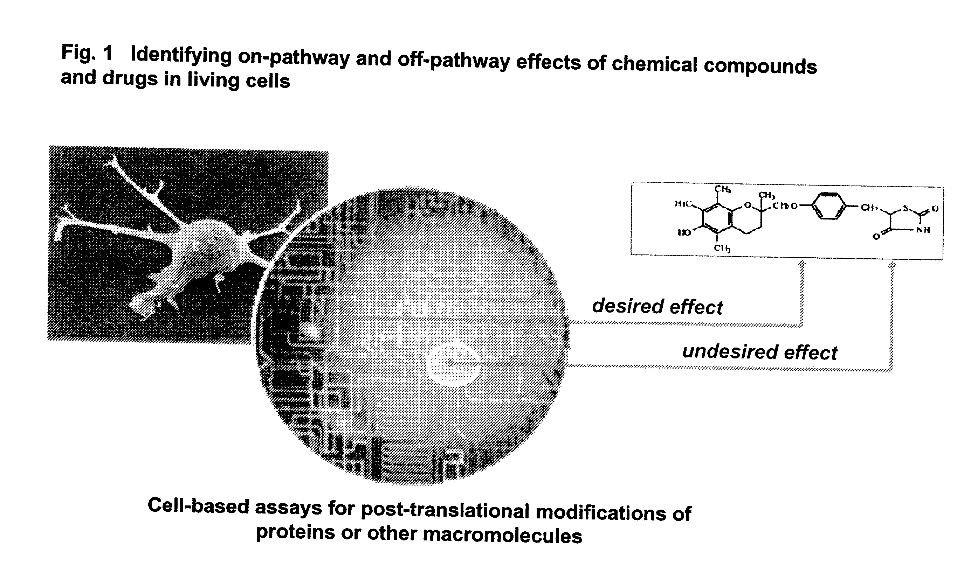

Figure 1 illustrates the objective of the present invention. The biochemical networks that control cellular behavior are represented as a circuit diagram. Drugs and chemical compounds have both known (intended) and unknown (unintended) effects within cells. Post-translational modifications of proteins and other molecules represent dynamic events that can be probed to identify known and unknown effects of drugs and lead compounds.
Figure 2 depicts the principle underlying the invention. The connectivity of cellular networks allows detection of the activity of a drug on a pathway, by measuring the effects of the drug on events 'downstream' of the drug target. Assays, representing post-translational modifications of proteins or other molecules, are shown in red. Drugs may either decrease or increase the post-translational modification status of a downstream protein or may alter its subcellular distribution. These changes can be measured in intact cells using immunofluorescence or other methods. Cross-talk between pathways can also be determined using this approach, for example, a drug acting on a first pathway may result in a change in modification status of a protein that participates in a second pathway.
Figure 3 depicts five key steps in pharmacological profiling according to the present invention. The results can be depicted in a variety of ways, for example, using a histogram; a matrix; a contour plot; or other suitable display method. In the matrix shown in Fig 3, green represents an increase in signal for a particular sentinel and red represents a decrease in signal. Such profiles are useful in comparisons, for example, in comparing a lead compound with a known drug or known toxicant or attrited compound.
Figure 4 shows the design of the proof-of-principle study for pharmacological profiling according to the present invention. Five different drugs were tested against three different pathways, resulting in pharmacological profiles consistent with their mechanisms of action.
Figure 5 shows representative photomicrographs, showing differential effects of forskolin, isoproterenol, anisomycin, or anisomycin + SB203580 on the subcellular localization and fluorescence intensity of phospho-CREB as assessed by immunofluorescence. A negative control well (secondary antibody only) is also shown.
Figure 6 shows representative photomicrographs, showing differential effects of EGF, EGF+PD98059, EGF+SB203580, and EGF+17AAG on the subcellular localization and fluorescence intensity of phospho-CREB as assessed by immunofluorescence.
Figure 7 shows differential effects of agents on the amount of phospho-CREB in the nucleus of human cells. Values are presented as a ratio relative to the untreated control
Figure 8 shows representative photomicrographs, showing differential effects of forskolin, isoproterenol, anisomycin, or anisomycin + SB203580 on the subcellular localization and fluorescence intensity of phospho-Hsp27 as assessed by immunofluorescence.
Figure 9 shows representative photomicrographs, showing differential effects of EGF, EGF+PD98059, EGF+SB203580, and EGF+17AAG on the subcellular localization and fluorescence intensity of phospho-Hsp27 as assessed by immunofluorescence.
Figure 10 shows differential effects of agents on the amount of phospho-Hsp27 in human cells. Results are presented as a ratio relative to the untreated control.
Figure 11 shows representative photomicrographs, showing differential effects of forskolin, isoproterenol, anisomycin, or anisomycin + SB203580 on the subcellular localization and fluorescence intensity of phospho-ERK as assessed by immunofluorescence.
Figure 12 shows representative photomicrographs, showing differential effects of EGF, EGF+PD98059, EGF+SB203580, and EGF+17AAG on the subcellular localization and fluorescence intensity of phospho-ERK as assessed by immunofluorescence.
Figure 13 shows differential effects of agents on the amount of phospho-ERK in human cells. Values are presented as a ratio relative to the untreated control.
Figure14 shows pharmacological profiles for the indicated drugs and biologic agents based on their activities on three pathways. Agents that act on the same pathway (e.g. isoproterenol and forskolin) produce similar profiles. Agents that act on different pathways produce different profiles (compare EGF vs. anisomycin; SB203580 vs. PD98059). Differences in potency (at the doses used) between agents acting on the same pathway (e.g. 17-AAG and PD98059) can also be seen.

### DETAILED DESCRIPTION OF THE INVENTION

### Identifying on-pathway and off-pathway effects of drugs (pharmacological profiling)

We sought to provide a method for drug discovery, that would be suitable for use on a large scale, and in particular to effect attrition of lead compounds with undesirable properties. In the process of making the present invention we tested three hypotheses. The first hypothesis was that quantitative, dynamic measurements of post-translational modifications of proteins within specific pathways would enable an assessment of the activation or inhibition of those pathways by a chemical compound or agent. The second hypothesis was that several types of dynamic events could occur in response to pathway activation: an increase or decrease in the amount of a modified protein, and/or the translocation of a modified protein from one subcellular compartment to another. The third hypothesis was that quantification and localization of the effects of drugs on a variety of individual, modified proteins within living cells would enable the development of profiles of drug activity. Pharmacological profiles could be used to identify compounds with desired profiles and to eliminate compounds with undesired profiles in the context of human biology.

Signal transduction networks are characterized by a high level of connectivity, and signals are transmitted in the context of extensive, dynamic protein complexes. To exploit this facet of cell biology to better understand drug action, we constructed assays for post-translationally-modified proteins. The assays enable probing the activity of specific signaling nodes under different conditions - time, drug concentration, pretreatment stimulus, etc. With this approach drug activity can be monitored at temporal and spatial levels within a network of pathways. By analyzing the response of diverse signaling nodes representing multiple target classes and pathways, we can define context-dependent drug activity and drug relationships.

By applying antibodies to fixed cells, one can measure the absolute levels of a particular protein or class of proteins, as well as specific post-translational modifications (e.g. phosphorylation, acetylation, ubiquitination) of a protein or class of proteins or other macromolecules. In making the present invention, cell-based assays using modification state-specific antibodies were used to monitor the dynamic association and dissociation of proteins in the absence or presence of chemical compounds. We created panels of quantitative, fluorescence assays for different phospho-proteins in live cells, and tested the activities of known drugs against the assay panels using automated microscopy. The intact, fixed cells can be analyzed by flow cytometry or by microscopy. Such methods can be automated, allowing assays to be performed in 96-well or 384-well plates. If automated microscopy is used, in combination with image analysis, the sub-cellular localization of a protein or modified protein (or class or proteins) can be assessed in this manner, enabling automated, "high-content" analyses. Flow cytometry and fluorescence spectroscopy can also be used for this purpose, where spatial resolution of the signal is not required. We demonstrate that the pattern of responses or "pharmacological profiles" detected by changes in intensity and/or physical location of the sentinel pair is related to the mechanism of action, specificity, and off-pathway effects of the drugs being tested; and that differences between drugs can readily be detected using this approach.

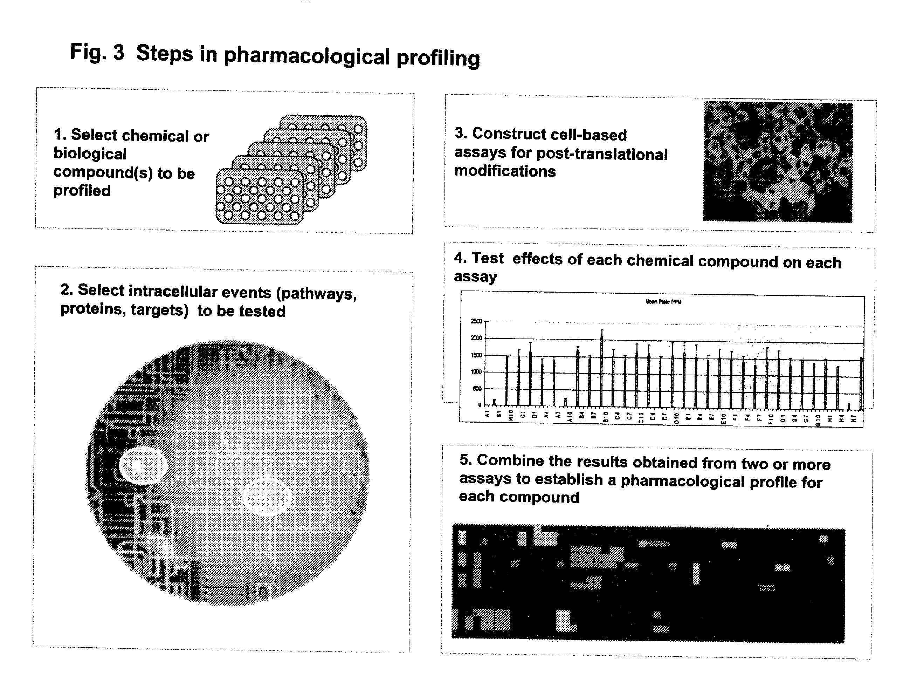

An overview of the invention is shown in Figure 3. Step 1 involves selecting the chemical compounds, drug candidates or drugs to be profiled. Step 2 involves selecting the proteins or other macromolecules to be included in the assay panel. The proteins can be identified, or selected, either rationally - for example, by prior knowledge of a pathway or a protein - or empirically. Moreover, an unlimited number of assays can simply be constructed at random and tested empirically for their responsiveness to any number of drugs or chemical compounds and the results combined into a pharmacological profile. Step 3 involves constructing the assays for post-translational modifications of macromolecules (proteins, DNA, etc). Such methods are well documented in the literature and can simply be adapted to the present invention if the proteins are appropriately selected, the antibody or probe is sufficiently specific, and the method is sensitive enough to detect and quantify changes in signal intensity or location due to the chemical compounds or drugs of interest. In step 4, each chemical compound or drug is tested against each assay at specific times and drug concentrations. Positive and negative controls are run for each assay, at each time point and stimulus condition. Each drug result is compared to a control (no treatment, or secondary antibody only) value. In step 5, the results of the assays are combined to establish a pharmacological profile for each compound. The resulting profiles can be displayed in a variety of ways. A simple histogram can be used to depict a pharmacological profile. Alternatively, the results of each screen are depicted in a color-coded matrix in which red denotes a decrease in signal intensity or location whereas green denotes an increase as shown here. Different shades of red and green can be used to depict the intensity of the change. A variety of visualization tools and third-party software can be used to display and analyze the profiles.

### EXPERIMENTAL PROTOCOL

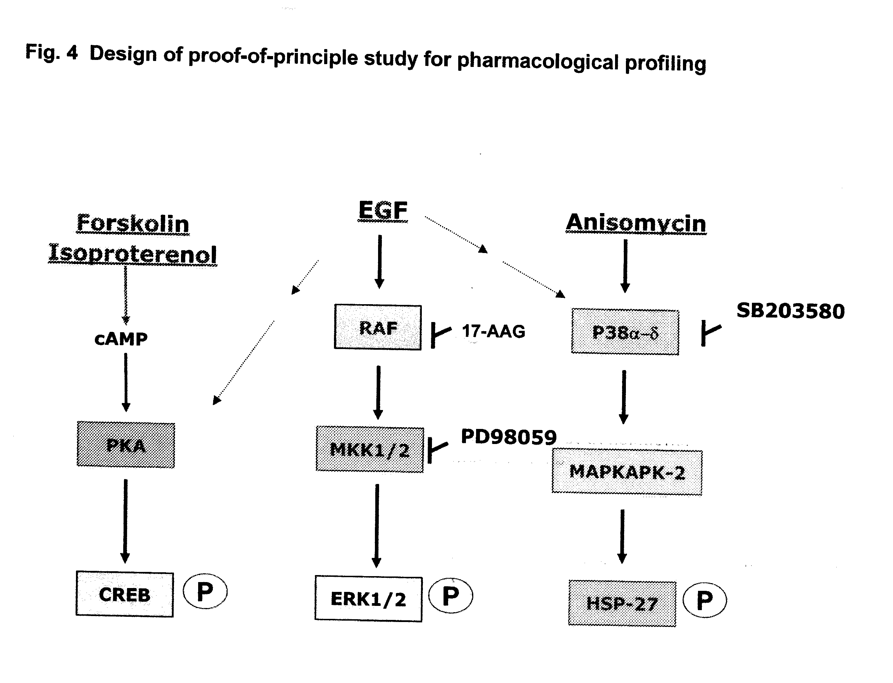

To demonstrate the general strategy and its application we studied multiple pathways that have been well-characterized in human cells. The experimental design is shown in Fig 4. For the proof of principle we used three canonical signal transduction pathways: the cyclic AMP-dependent pathway; the ERK mitogen-activated protein kinase (MAPK) pathway; and the p38/MAPKAPK2 pathway. Each pathway has many other steps that have been documented in the biochemical literature; the diagram shows only a select few of the many proteins that participate in each pathway.

Pathway 1: The beta-adrenergic receptor has been well characterized as a result of its pharmacological importance. This G-protein-coupled receptor (GPCR) is coupled to adenylyl cyclase via the small GTP-binding protein, Gₛ. Binding of isoproterenol or other beta-adrenergic agonists to this receptor leads to activation of adenylate cyclase. When adenylyl cyclase is activated, it catalyses the conversion of ATP to cyclic AMP, which leads to an increase in intracellular levels of cyclic AMP. Cyclic AMP (cAMP) is a second messenger that activates the cyclic AMP-dependent protein kinase known as protein kinase A (PKA). Levels of cAMP are controlled through the regulation of the production of cAMP by adenylate cyclase, and the destruction of cAMP by phosphodiesterases. Adenylate cyclase can also be activated directly by agents such as forskolin, a diterpene that is widely used in studies aimed at dissecting intracellular signalling pathways. One of the best characterized substrates for PKA is the transcription factor CREB which is phosphorylated on serine133 (S133) in response to adrenergic agonists or other activators of PKA. Phosphorylation of CREB has been shown to increase its transcriptional activity for its target genes (Montminny et al). Therefore both forskolin and isoproterenol would be expected to activate steps that are downstream of PKA in living cells, including the phosphorylation of CREB. They should have similar pharmacological profiles based on their known activities.

Pathway 2: ERK/MAPKs are key relay points in the transmission of growth factor-generated signals. This canonical growth factor receptor-stimulated pathway is initiated by a cell surface receptor, such as the epidermal growth factor (EGF) receptor tyrosine kinase. Activated EGF receptors bind to adaptor proteins and guanine nucleotide exchange factors, such as the protein SOS. SOS, in turn, activates small GTPases such as Ras, which then lead to phosphorylation and activation of a cascade of kinases including B-Raf and ERKs. By measuring the activity of a distal step in the pathway, such as phosphorylation of ERKs, the activity of upstream steps can be inferred. We profiled two different agents, PD98059 and 17-AAG, against this pathway. PD98059, a known inhibitor of the protein kinase known as MEK (MKK1/2), blocks events downstream of its target ncluding the transcription factors ERK (shown in Fig. 4) and ELK. 17-AAG (17-allylamino-17-demethoxygeldanamycin) is an ansamycin antibiotic that is currently in clinical trials for the treatment of cancer. 17-AAG binds to a highly conserved pocket in the Hsp90 chaperone protein and inhibits its function. Hsp90 is required for the refolding of proteins during cellular stress, and for the conformational maturation of a subset of signaling proteins. Treatment of cells with 17-AAG causes the proteasomal degradation of Hsp90 client proteins, which include RAF, AKT and HER2. Given (a) a sufficiently specific anti-phospho-ERK antibody; (b) a cell type that is responsive to EGF; and (c) a sufficient quantity of PD98058; and (d) an immunofluorescence method that is capable of detecting phospho-ERK in intact cells, it should be possible to determine the effects of PD98059 and 17-AAG on the amount and/or location of phospho-ERK in living cells. PD98058 is a relatively selective kinase inhibitor whereas 17-AAG affects a broad spectrum of Hsp90 client proteins. Therefore both agents would be expected to reduce the effect of EGF on phosphor-ERK but would have disparate effects on other pathway sentinels, for example Pathway 3.

Pathway 3: The p38 serine/threonine protein kinase is the most well-characterized member of the MAP kinase family. It is activated in response to inflammatory cytokines, endotoxins, and osmotic stress. It shares about 50% homology with the ERKs. The upstream steps in activation of the cascade are not well defined. However, downstream activation of p38 occurs following its phosphorylation (at the TGY motif) by MKK3, a dual specificity kinase. Following its activation, p38 phosphorylates MAPKAPK2, which in turn phosphorylates and activates heat-shock proteins inclulding HSP27. Anisomycin is a natural product that has been shown to activate stress related pathways in cells, including the p38 pathway shown in Fig 4. SB203580 [4-(4-fluorophenyl)-2-(4-methylsulfinylphenyl)-5-(4-pyridyl)1H-imidazole] is a very specific inhibitor of p3 mitogen-activated protein kinase (MAPK) and is widely used as a tool to probe p38 MAPK function in vitro and in vivo. If anisomycin is specific for the p38 pathway in these cells, anisomycin would increase phospho-Hsp27 but would have no effect on phospho-CREB or phospho-ERK. The p38-specific inhibitor, SB203580, would be expected to block the effects of anisomycin on Hsp27. Therefore, given a suitable anti-phospho-Hsp27 antibody, we would expect to see an increase in phosphorylation of Hsp27 in response to pathway activation by anisomycin in living cells. This effect should be blocked by SB203580.

We assessed the effects of the above-mentioned compounds on the three pathways and used the results to construct pharmacological profiles for the agents. Specifically we assessed changes in the phosphorylation status of the pathway 'sentinels' by constructing high-content, immunofluorescence assays using phospho-specific antibodies targeted to the downstream proteins in the pathways of interest. Human cells (HEK293) were treated with drugs and the phosphorylation status of the three downstream proteins was assessed in the absence or presence of epidermal growth factor (EGF). Cells were then fixed and probed with antisera generated against the phosphorylated forms of CREB (S133), ERK1/2 (phospho T*EY*), or phospho Hsp27 (S78/S82). The ERK1/2 antibodies specifically recognize the MAPK/ERK1 and MAPK/ERK2 protein kinases only when they are phosphorylated on Threonine 202 and Tyrosine 204 in the activation loop. Phosphorylation of these amino acids has been shown to be necessary and sufficient for kinase activation, and therefore is a surrogate marker for activation of the kinases. Changes in the level and sub-cellular localization of a phosphorylated protein following treatment with a drug would indicate a functional connection between the drug and the pathway of interest.

Details of the methods used are as follows. HEK293T cells were seeded in black-walled, poly-lysine coated 96-well plates (Greiner) at a density of 30,000/well. After 24 hours, cells in duplicate wells were treated with combinations of different drugs and stimulus as follows: (a) 2 micromolar isoproterenol or 1 micromolar forskolin for 15 min.; (b) 25 micromolar SB203580 or vehicle (DMSO) for 90 minutes and 10 micrograms/ml anisomycin added to the cells during the last 10 min.; (c) 20 microolar PD98059, 25 micromolar SB203580, 5 microM 17-AAG or vehicle alone for 90 min. (d) as for (c), but with 100ng/ml hEGF added to the cells during the last 5 min of drug treatment. The drugs were purchased from Calbiochem and hEGF was from Roche. Four sets of cells treated as described were prepared. The cells were rinsed once with PBS and fixed with 4% formaldehyde for 10 min. The cells were subsequently permeabilized with 0.25% Triton X-100 in PBS and incubated with 3% BSA for 30 min to block non-specific antibody binding. Each of the 4 sets of identically treated cells were then incubated with rabbit antibodies against phosphorylated CREB (Ser133), Hsp27 (Ser82), or pERK (T202/Y204) (Cell Signaling Technology, Inc.). Control wells were incubated with bovine serum albumin (BSA) in PBS. The cells were rinsed with PBS and incubated with Alexa488 conjugated goat anti-rabbit secondary antibody (Molecular Probes). Cell nuclei were stained with Hoechst33342 (Molecular Probes).

Images were acquired using Discovery-1 High Content Imaging System (Molecular Devices). Background fluorescence due to nonspecific binding by the secondary antibody was established with the use of cells that were incubated with BSA/PBS and without primary antibodies.

Raw images in 16-bit grayscale TIFF format were analyzed using ImageJ API/library (http://rsb.info.nih.gov/ij/, NIH, MD). First, images from the fluorescence channels (Hoechst and Alexa 488) were normalized using the ImageJ built-in rolling-ball algorithm [S.R. Sternberg, Biomedical image processing. Computer, 16(1), January 1983]. Next a threshold was established to separate the foreground from background. An iterative algorithm based on Particle Analyzer from ImageJ is applied to the thresholded Hoechst channel image (HI) to obtain the total cell count. The nuclear region of a cell (nuclear mask) is also derived from the thresholded HI. The positive particle mask is generated from the thresholded Alexa 488 image (YI). To calculate the global background (gBG), a histogram was obtained from the unthresholded Alexa signaland the pixel intensity of the lowest intensity peak was identified as gBG. The Hoechst mask and Alexa mask are overlapped to define the correlated sub-regions of the cell. All means were corrected for the corresponding gBG. For each set of experiments (assay + drug treatment + treatment time), fluorescent particles from eight images were pooled. For each parameter, an outlier filter was applied to filter out those particles falling outside the range (mean ± 3SD) of the group. Finally the sample mean or control mean for each parameter was obtained from each filtered group. Results for drug treatments were normalized to the control for each experiment.

### RESULTS

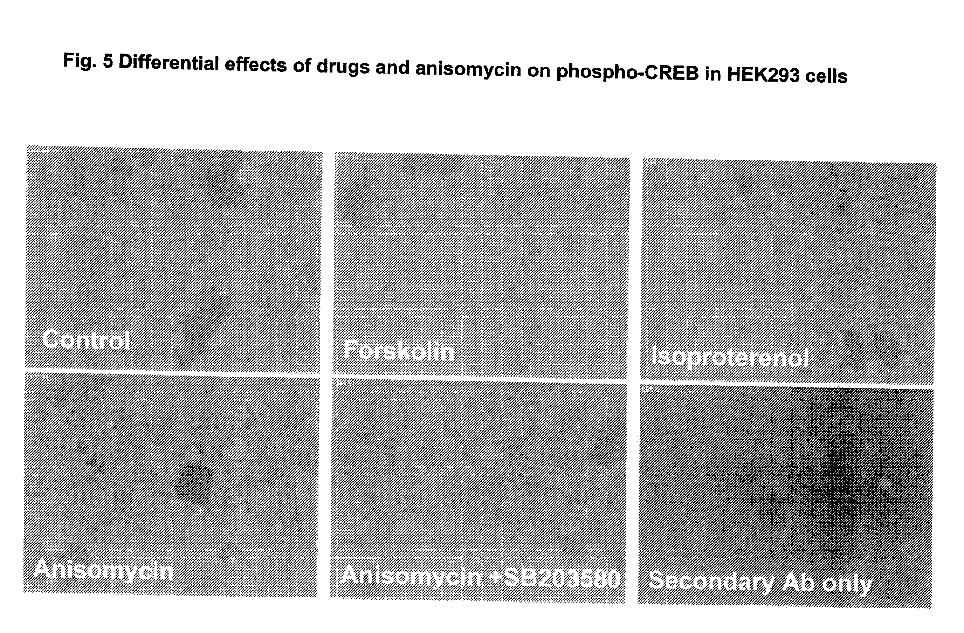

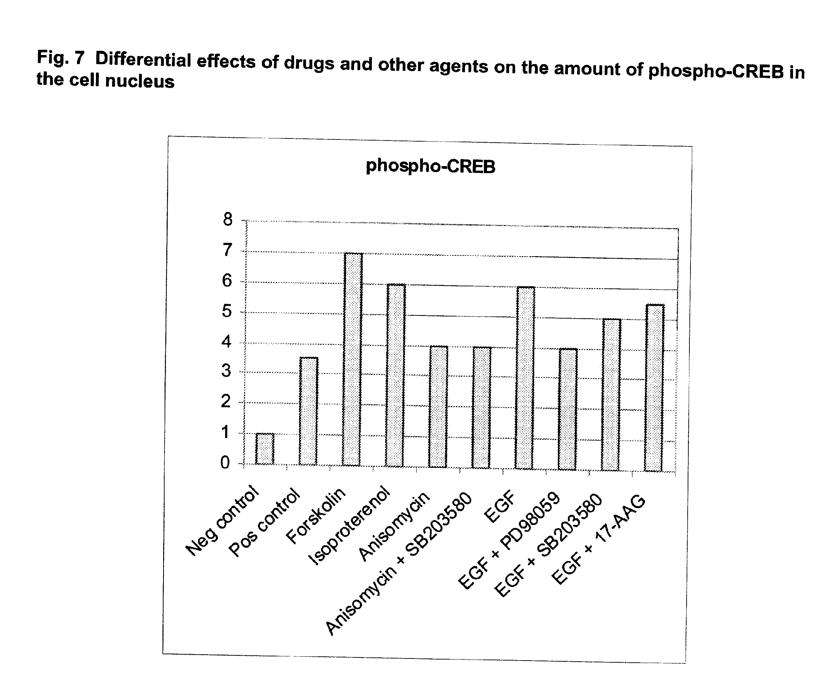

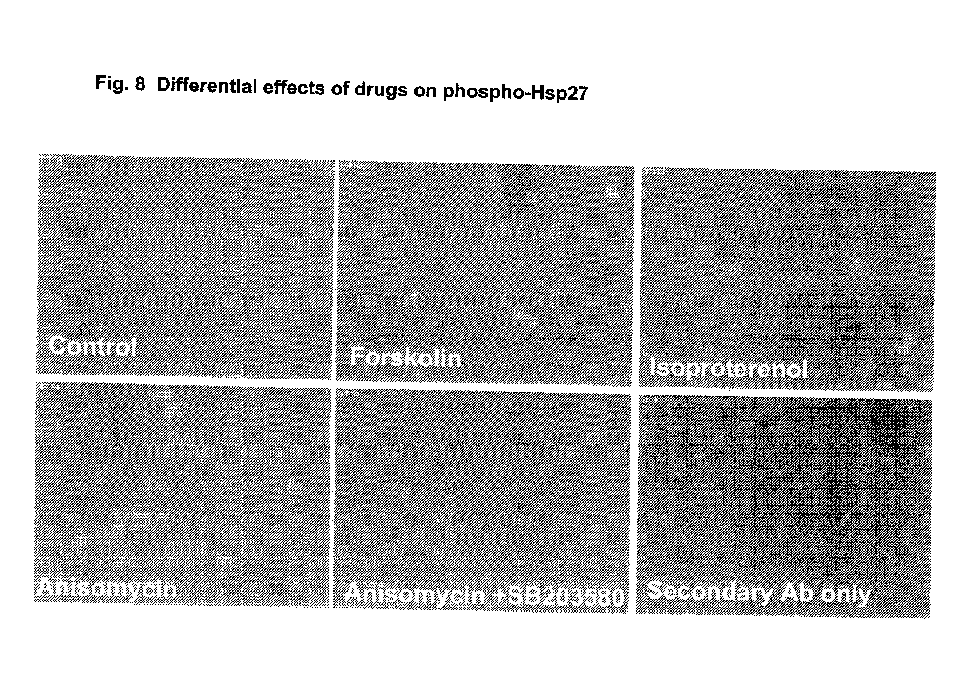

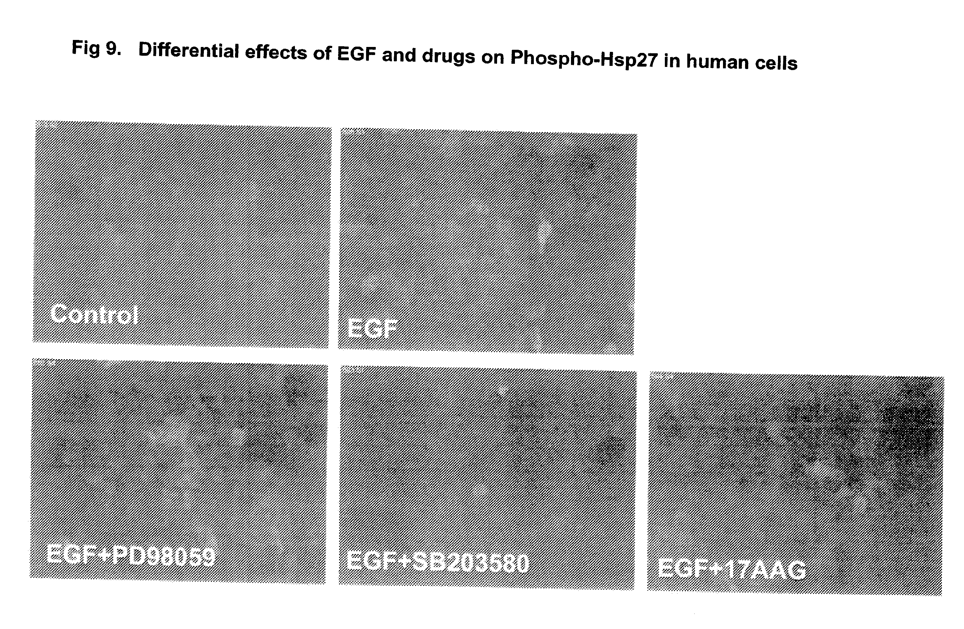

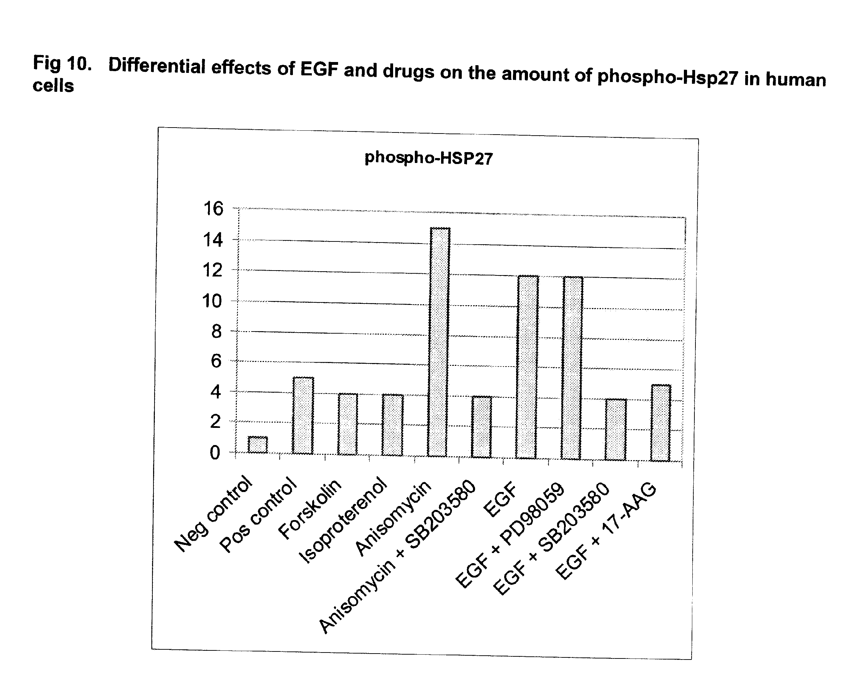

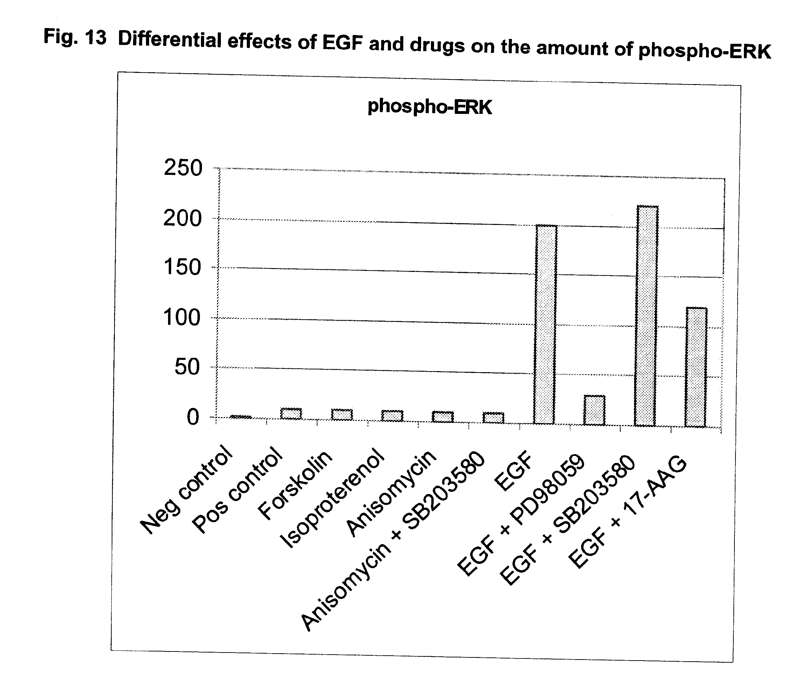

Results of the experiments are shown in Figs 5-14, starting with the cyclic AMP-dependent (CREB) pathway (Fig 5). The negative control wells (lower left) showed little or no signal with secondary antibody alone, demonstrating that the detection of phospho-CREB was accomplished with the phospho-specific antibody. In the presence of CREB phospho-specific antibody there was a clear fluorescence signal (control, upper left) that was localized predominantly at in a membrane/perinuclear pattern. As assessed by immunofluorescence, forskolin and isoproterenol both increased the phosphorylation of CREB and changed its subcellular distribution to a predominantly nuclear pattern, relative to the control (untreated) cells. These effects could be seen clearly in the fluorescence micrographs (Fig 5, upper panels). In contrast, anisomycin had little or no effect on the intensity or the subcellular location of phospho-CREB, demonstrating a lack of connectivity between the anisomycin-dependent pathway and the CREB pathway.

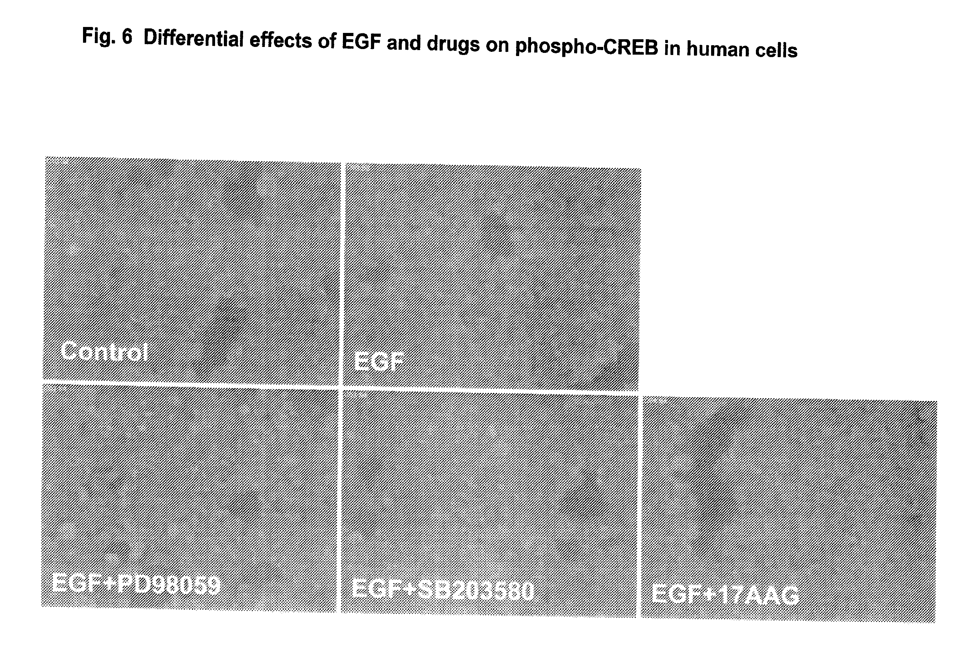

As shown in Fig 6, EGF also induced the formation of phospho-CREB. The effects of EGF on phospho-CREB are consistent with cross-talk between the EGF-dependent and cyclic AMP-dependent pathways as depicted in Fig 4. The effect of EGF was reduced by PD98059, suggesting either that the PD compound has an off-pathway effect on the CREB pathway, or that the cross-talk between the EGF and CREB pathways occurs at a level below MEK (the target of PD98059). These results indicate that both direct and indirect effects of agonists and drugs on pathways can be assessed by assays of events downstream of the point of action of the agonist or drug, substantiating the premise that the connectivity of cellular networks can be exploited for use in identifying the spectrum of drug activities. The results also demonstrate the ability of the methodology to differentiate between agents that activate or inhibit pathways and those that have no effects on those pathways.

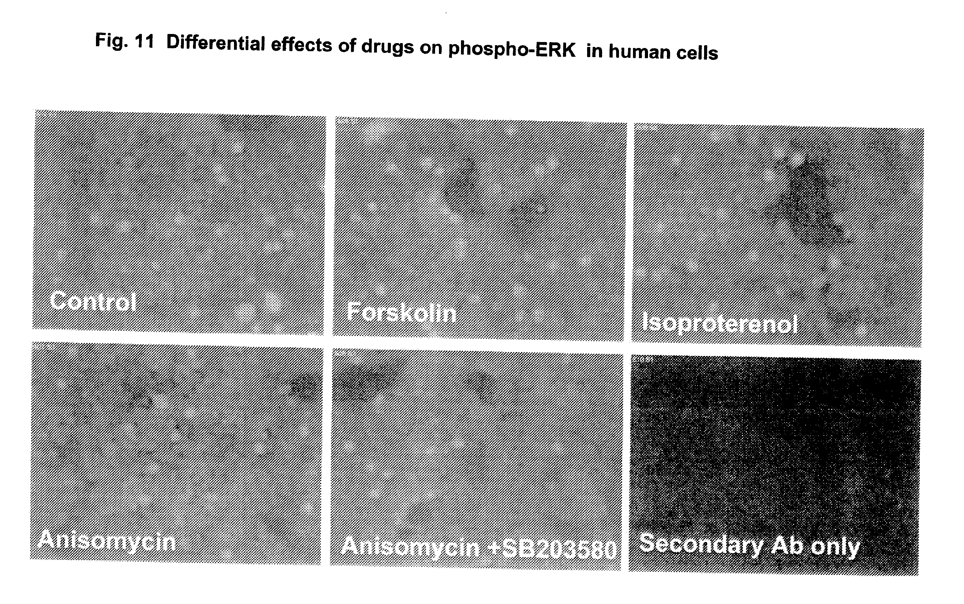

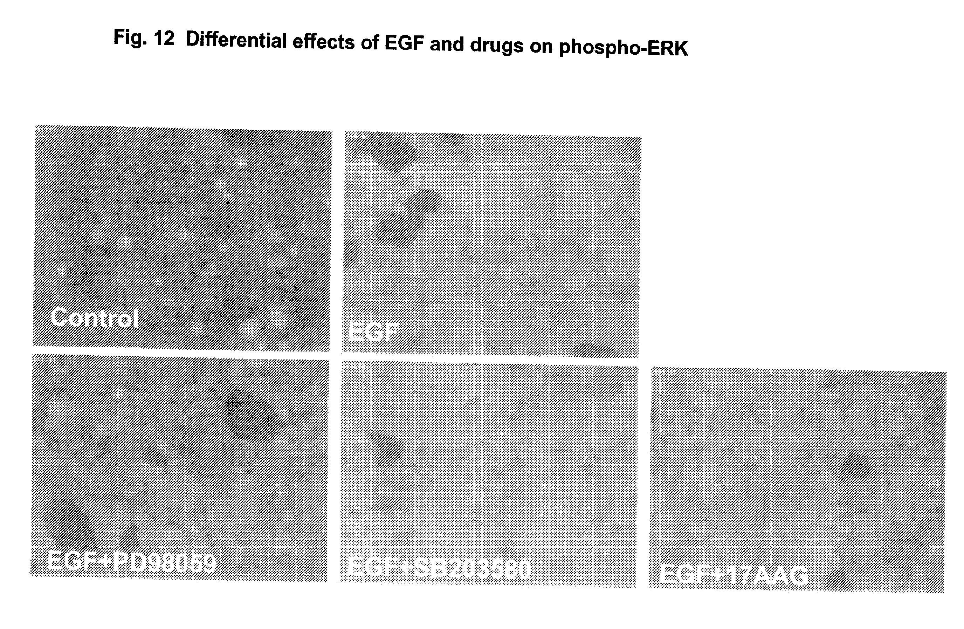

Differential activities of drugs on their expected targets/pathways were also observed. For example, EGF strongly stimulated the MAP kinase pathway, as expected, resulting in highly induced levels of ERK/MAP kinase phosphorylation (Fig 12-13). Forskolin, isoproterenol, and anisomycin had no effects on this pathway (Fig 11). The compound PD98059, a known inhibitor of the kinase MEK, significantly blocked the phosphorylation of ERK in response to EGF, as expected. 17-AAG was also effective at reducing the effects of EGF on ERK. On the other hand, treatment of these cells with the p38-specific inhibitor SB203580 has no effect on EGF-stimulated ERK phosphorylation since SB203580 selectively acts on a pathway that is not connected to ERK. The results demonstrate the ability of the methodology to pinpoint on-pathway effects of drugs and to assess drug selectivity against pathways in human cells.

This strategy also reveals cross-talk between pathways. Anisomycin induced the p38 pathway as assessed by increases in phospho-Hsp27 in anisomycin-treated cells. Since anisomycin had no effect on the CREB or ERK pathways, it was quite selective for the p38 pathway. SB203580 completely blocked the effects of anisomycin on Hsp27, consistent with the known mechanism of action of the SB inhibitor. EGF also elicited p38 pathway activation (resulting in HSP27 phosphorylation), and this response was blocked by the p38 inhibitor SB203580, demonstrating cross-talk between the EGF and p38 pathways at a level upstream of the site of action of SB203580. In contrast the MEK inhibitor PD98059 had no effect on EGF-induced Hsp27 phosphorylation, showing that PD98059 was selective for the MEK/ERK pathway.

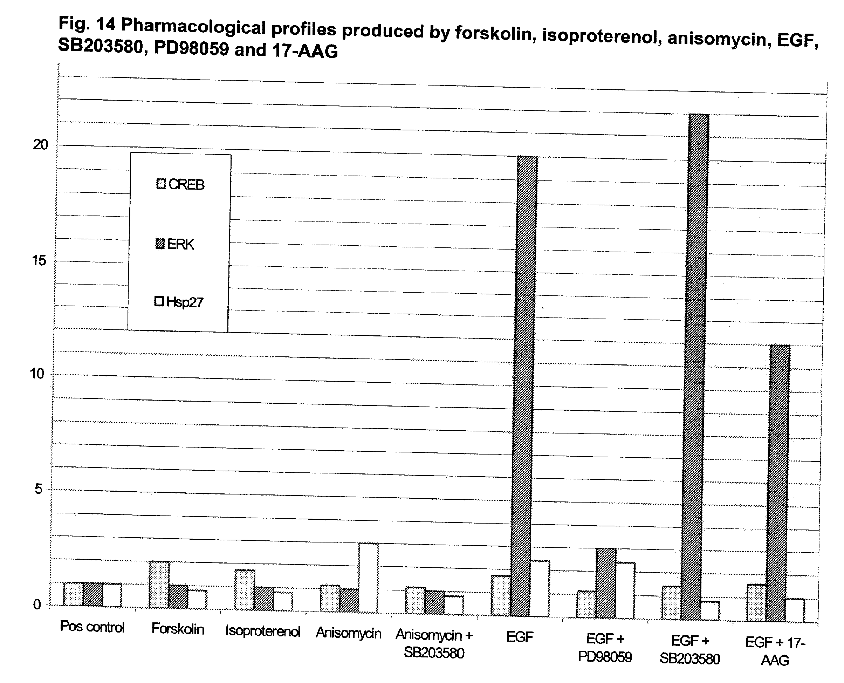

The pharmacological profiles depicted in Fig 14 demonstrate the similarities and differences between the agents. These pharmacological profiles can be used as fingerprints for drugs with certain mechanisms of action and selectivity. The fingerprints can be used to identify novel compounds with desired cellular effects and to eliminate compounds with undesired cellular effects. For example, using these methods, novel agents can be identified with cellular effects similar to EGF, to anisomycin, or to one of the kinase inhibitors. Establishing profiles for agents with known toxic or adverse effects will allow for attrition of novel compounds with similar (toxic or adverse) profiles. As the assay panels expand they will become ever more predictive. Profiling of known drugs, failed compounds and toxic agents will enable the development of fingerprints of drugs with established clinical outcomes. As the panels expand they will enable the development of drugs with very specific safety and efficacy profiles.

### Scope of the invention

It will be understood by one skilled in the art that the present invention is not limited to the exact pathway, assay sentinel, assay protocol, detection method, or to particular instrumentation or software. The present invention teaches that cell-based fluorescence or luminescence assay panels, and in particular immunofluorescence assays, can be used for pharmacological profiling of drugs, biologic agents, natural products, and other compounds of interest.

There is virtually no limit on the types, numbers, or identities of the proteins or assay reagents that can be used in conjunction with this invention. There are likely to be thousands of post-translational modifications of proteins that occur in mammalian cells. These will be either constitutive or dynamic; and either redundant or non-redundant. Dynamic (responsive), non-redundant assays will be the most useful for pharmacological profiling as they will respond to pathway perturbations. Fortunately, one can determine empirically whether a specific protein or other macromolecule is useful in profiling, by simply constructing an assay for the modification and testing it for responsiveness against a range of drugs, gene annotation reagents -such as siRNA - or other compounds. A non-redundant assay is one that provides distinct information, beyond the information provided by any other assay. As the pathways regulating cellular function are gradually elucidated it will eventually be possible to construct a completely predictive assay panel based on the methods provided herein. It will be possible to determine whether the panel is predictive by comparing the profiles of well-characterized agents that cause particular adverse effects in animals or in man, with the profiles of agents that do not cause the same effects. Such a panel would enable testing of any compound to determine its spectrum of activities and to determine any off-pathway activities suggestive of adverse consequences. The advantage of the approach is that it can be performed in high throughput such that thousands of lead compounds can be tested, prior to clinical studies, allowing early attrition of compounds with undesirable profiles.

The informativeness of the approach is based not on the number of proteins assayed but on the breadth of pathways covered. Adding more sentinels into the same pathways will help in defining novel mechanisms of action and in identifying potential new drug targets; but will not necessarily provide additional predictive power. Ultimately, a single informative sentinel for each cellular pathway is needed. A completely predictive platform might be achieved with 200-500 assays. These calculations are speculative, but may help to explain our predictions. The biochemical literature, and our own experience, suggests that biochemical networks are highly ramified. For example, in the process of mapping interactions among human proteins, we identified an average of 5 interactions per protein; a number that is consistent with protein interaction maps of model organisms such as yeast. If one assumes 30,000 proteins in the human proteome (excluding splice variants, that is) then there may be around 6000 human protein-protein interactions that are physically separated by one or more degrees of separation (30,000/5). Finally, if we assume that each of 6000 non-redundant sentinels serves to report on the activity of 15 upstream events, then a collection of 400 sentinels would report out the activity of every pathway in the cell.

The present invention is not limited to the measurement of modifications of individual proteins. Cellular assays that can be used to quantify or localize protein-protein interactions can be included in such panels. Suitable methodology for such measurements includes fluorescence resonance energy transfer (FRET), bioluminescence resonance energy transfer (BRET), protein-fragment complementation assays (PCA) and enzyme-fragment complementation assays (beta-galactosidase complementation). Cellular assays that can be used to construct assay panels for pharmacological profiling can include pan-cellular measurements as well as measurements of individual proteins. For example, the overall level of tyrosine phosphorylation of cellular proteins (as assessed with pan-phosphotyrosine antibodies) can be used to assess on-pathway and off-pathway effects of known and novel compounds and to build pharmacological profiles. Measurements of particular motifs (ubiquitin etc.) will also be useful for the construction of the assay panel as they provide an overall assessment of cellular metabolic and phenotypic status. Overall and specific cellular protease activity can be assessed by loss of an epitope upon proteolysis, resulting in a reduction in signal as assessed with an epitope-specific antibody. Antibodies that discriminate GTP vs GDP-bound proteins such as G proteins coupled to GPCRs could be developed and used to assess G protein status as a component of cell signaling. Splice variants or isoforms of a particular protein could also be measured - e.g. with the aid of an antibody that only recognizes cleaved form of a sentinel protein. Such assays would indicate the state of apoptosis in the cell. In addition we will use antibodies that discriminate between splice variants of particular kinases - MKK3 vs. MKK1/2. These agents can also be combined in the same assay; for example a phospho-specific anti-BAD antibody could be combined with a pan-AKT antibody to simultaneously assess the two key regulators of apoptotic pathways. Measurements of histone acetylation (with acetyl-specific anti-histone antibodies) would enable an assessment of the overall balance between acetylation and deacetylation, a key regulator of gene transcription. As mentioned above, any such pathway measures or cellular indicators can be combined with cellular stains to increase the informativeness of the assay panels. Dyes capable of measuring membrane potential can also be useful in such an assay panel. For example, stains for mitochondrial membrane potential can be used to distinguish between drugs with different cellular effects and to construct pharmacological profiles in conjunction with this invention.

In addition to proteins, a variety of macromolecules are modified post-translationally, including DNA and lipids. Methylation of DNA is important in the sequence-specific and gene-specific regulation of transcription. Phosphorylation of lipids is important in the control of cell signaling; for example, the balance between inositol polyphosphates is crucial in regulating the level of the second messenger, inositol trisphosphate (IP3); and the fatty acid composition of phospholipids such as phosphatidylcholine, phosphatidylinositol and phosphatidylserine regulates membrane fluidity and permeability. As the toolbox of modification-state-specific reagent expands, such assays will be added into the panels we are constructing for pharmacological profiling.

The entire contents including the references cited therein of the following patents and publications are incorporated by reference in their entirety for all purposes to the same extent as if each individual patent, patent application or publication were so individually denoted.
US 6,372,431 Cunningham, et al.
US 6,801,859 Friend, et al.
US 6,673,554 Kauvar, et al.
US 6,270,964 Michnick, et al.
US 6,294,330 Michnick, et al.
US 6,428,951 Michnick, et al.
US Patent Application 20030108869 Michnick, et al.
US Patent Application 20020064769 Michnick, et al.
Nielsen et al., PNAS 100: 9330-9335 (2003)

Although the present invention has been described with reference to specific details of certain embodiments thereof, it is not intended that such detail should be regarded as limitations upon the scope of the invention, except as and to the extent that they are included in the accompanying claims.

## Claims

1. A method for performing pharmacological profiling of a chemical compound, said method comprising (a) constructing a panel of cell-based assays, wherein said assays comprise the amount and/or post-translational modifications of two or more macromolecules; (b) contacting said cells with said chemical compound; (c) measuring the amount and/or the subcellular location of the signals in said cells with said cell-based assays; (d) using the results of (c) to construct a pharmacological profile for said compound.

2. The method of claim 1, wherein said chemical compound comprises chemical structures and drug targets, wherein said drug targets are selected from the group consisting of G-protein coupled receptors, growth factor receptors, protease inhibitors, nuclear hormone receptors, membrane hormone receptors, kinases, phosphatases, hydrolases and proteasome inhibitors.

3. The method according to claim 1 or 2, wherein said cell type of said cell-based assay is a human cell, a mammalian cell, a plant protoplast, yeast or fungus.

4. The method according any of claims 1-3, wherein said post-translational modifications include methylation, acetylation, farnesylation, glycosylation, myristylation, ubiquitination and sumoylation.

5. The method according to claim 4, wherein said post-translational modification are detected using antibodies in conjunction with immunofluorescence.

6. The method according any of claims 1-5, wherein said macromolecules are proteins.

7. The method according to any of claims 1-6, wherein said amount of the signal is determined by the quantification of a bulk fluorescent or luminescent signal.

8. The method according to any of claims 1-6, wherein said subcellular location of the signal is imaged by automated microscopy or image analysis and is detected and quantified.
